# EUROPÄISCHE PATENTSCHRIFT

(11) **EP 2 085 147 B1**
(45) Veröffentlichungstag und Bekanntmachung des Hinweises auf die Patenterteilung: **04.04.2012**
(21) Anmeldenummer: 09405004.4
(22) Anmeldetag: 12.01.2009
(51) Int. Cl.: A61M 5/145, B05C 17/01, A61M 5/20

(54) **Vorrichtung mit Druck beaufschlagtem Kolben zum Austragen einer Mehrfachspritze oder Mehrfachkartusche**
Device with pressure charged piston for dispensing a multiple syringe or multiple cartridge
Dispositif doté d'un piston sur lequel est appliquée une pression, destiné à vider une seringue multiple ou une cartouche multiple

(30) Priorität: 29.01.2008 CH 1242008
(43) Veröffentlichungstag der Anmeldung: 05.08.2009
(73) Patentinhaber: Medmix Systems AG, 6343 Rotkreuz (CH)
(72) Erfinder: Keller, Wilhelm A., 6402 Merlischachen (CH)
(74) Vertreter: Detken, Andreas

(56) Entgegenhaltungen:
- WO-A-95/03844
- WO-A-99/37343
- DE-A1- 1 957 833
- DE-A1-102005 048 871
- DE-U1- 20 006 986
- US-A- 2 565 081
- US-A- 3 702 608
- US-B1- 6 228 067

## Beschreibung

Die Erfindung betrifft eine Vorrichtung zur Aufnahme und zum Austragen einer Spritze gemäss Oberbegriff von Patentanspruch 1.

Eine solche Vorrichtung zum Austragen einer Spritze mit Injektionsnadel ist aus der US-A-2 565 081 bekannt, wobei die Vorrichtung zwei teleskopartig ineinanderschiebbare Teile zur Anpassung an das Austragvolumen aufweist und die Einkomponenten-Spritze verschiebbar im vorderen Teil geführt ist. Der Austragkolben ist mit einer Druckfeder beaufschlagt und verschiebt unter Druckeinwirkung die Spritze und ist an seinem hinteren Ende durch einen Arretierzahn gespannt gehalten. Dadurch, dass die Spritze innerhalb des Halter verschiebbar ist, muss diese dem Halter angepasst und relativ lose gehalten sein.

US-A-3 702 608 offenbart eine Austraganordnung mit einem Teil zur Aufnahme einer Einfachspritze am dem Auslass entgegengesetzten Ende, wobei sowohl die Spritze als auch ihr Stössel verschiebbar angeordnet sind und die Anordnung mehr als eine Druckfeder aufweist.

WO 99/37343 offenbart ein Injektionsgerät, das ausgebildet ist, eine Einkomponentenspritze verschiebbar aufzunehmen und einen relativ komplizierten Auslösemechanismus aufweist.

Bei der Wundversorgung finden neben herkömmlichen Verbandmaterialien zur keimfreien Wundabdeckung auch Flüssigkeiten, Pasten und Gelatine Verwendung, welche in Kombination mit anderen Stoffen für eine Wundversiegelung, Wundheilung und Schmerzbekämpfung sorgen. Dabei kommen in zunehmendem Masse Mehrkomponenten-Stoffe zum Einsatz, bei welchen jedoch vor dem Auftragen auf die Wunde ein Zusammenbringen und Vermischen erforderlich ist. Hierzu werden die Komponenten beispielsweise in einer Doppelspritze gelagert und vor der Applikation auf die Haut- oder Wundoberfläche mit Hilfe eines statischen Mischers oder Sprühsystems gemischt. Das Austragen des Gemisches erfolgt durch eine manuelle Druckeinwirkung auf den Stössel der Doppelspritze.

Insbesondere bei Langzeitpatienten oder bei chronischen Wunden ist eine selbstständige Wundversorgung vom Patienten nicht nur wünschenswert sondern auch kostendämpfend. Jedoch erfordert der Austragvorgang bei einer herkömmlichen Doppelspritze in nicht zu unterschätzendem Masse eine Kraft und eine gewisse manuelle Geschicklichkeit seitens des Patienten. Je nach Stelle der Wunde kann das Bedienen der Doppelspritze durch den Patienten Schwierigkeiten bereiten.

Auch ist eine Dosiermöglichkeit der auszutragenden Komponenten wünschenswert, welche durch herkömmliche Doppelspritzen nur unzureichend gewährleistet ist.

Hiervon ausgehend liegt der Erfindung die Aufgabe zugrunde, eine Vorrichtung der eingangs genannten Art derart weiterzubilden, dass sie eine Verwendung von Mehrfachspritzen erlaubt, kostengünstig herstellbar ist und eine verbesserte Handhabbarkeit für Patienten gewährleistet.

Es ist eine weitere Aufgabe der Erfindung, eine wiederholte Verwendung und ein genaues Dosieren beliebiger Austragmengen der Vorrichtung zu ermöglichen.

Diese Aufgaben werden durch eine Vorrichtung gemäss dem unabhängigen Patentanspruch 1 gelöst. Weitere bevorzugte Ausführungsformen der Erfindung sind in den abhängigen Patentansprüchen definiert.

Nachfolgend wird die Erfindung anhand von Zeichnungen von Ausführungsbeispielen näher erläutert. Dabei zeigen:

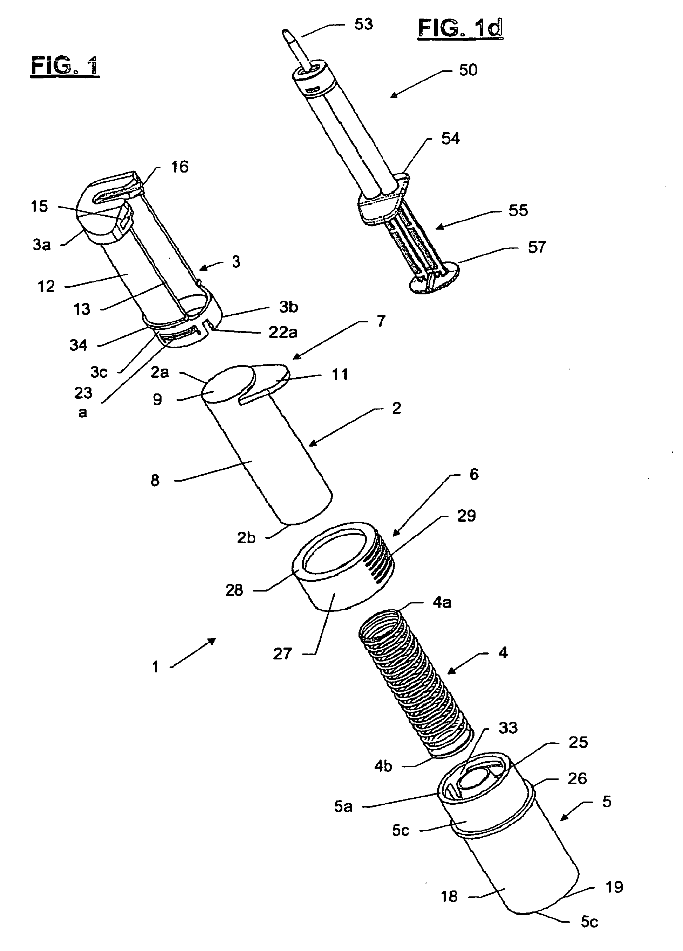

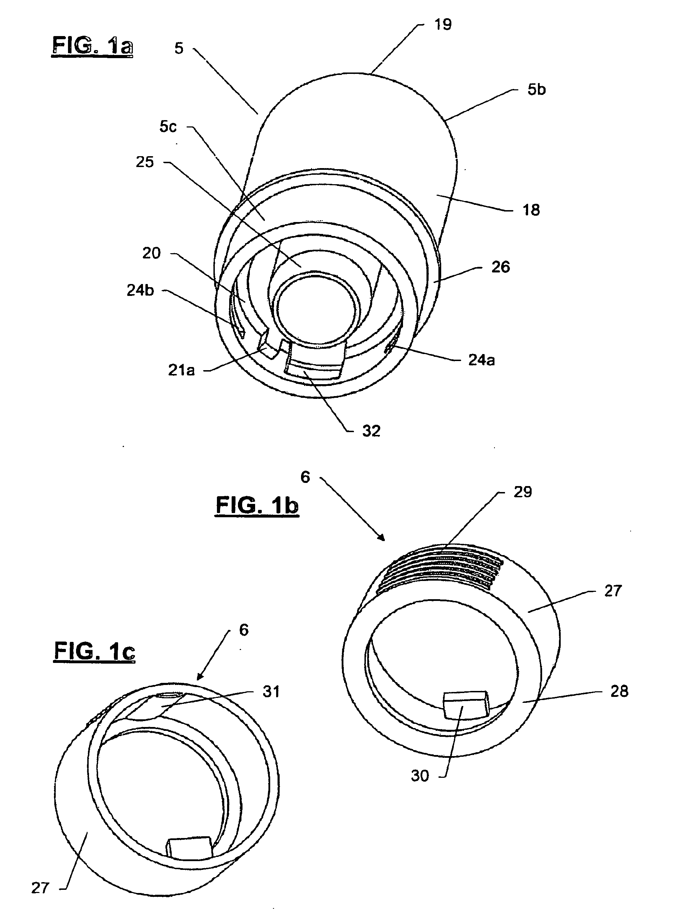

- Fig. 1: eine Explosionsdarstellung eines ersten Ausführungsbeispiels einer erfindungsgemässen Vorrichtung zum automatisierten Austragen einer Doppelspritze;
- Fig. 1d: eine perspektivische Ansicht der zusammengebauten Vorrichtung gemäss Fig. 1,
- Fig. 1a: eine perspektivische Ansicht des Handhabungsteils der in Fig. 1 gezeigten Vorrichtung,
- Fig. 1b: eine perspektivische Ansicht der Arretierhülse der in Fig. 1 gezeigten Vorrichtung,
- Fig. 1c: eine andere perspektivische Ansicht der Arretierhülse von Fig. 1b,
- Fig. 2:: eine perspektivische Ansicht eines Teils der in Fig. 1 gezeigten Vorrichtung mit dem Austragkolben in gespannter Position
- Fig. 3: eine perspektivische Ansicht des Austragkolbens von Fig. 1,
- Fig. 4:: eine teilweise Schnittansicht gemäss der Ebene IV- IV in Fig. 2, in welcher die Arretierstellung des Austragkolbens in der gespannten Kolbenposition dargestellt ist;
- Fig. 5:: eine teilweise Schnittansicht gemäss der Ebene V-V in Fig. 2, in welcher die gelöste Stellung der Arretierhülse dargestellt ist;
- Fig. 6:: eine perspektivische Ansicht eines zweiten Ausführungsbeispiels einer erfindungsgemässen Vorrichtung mit einer Doppelspritze zum automatisierten Austragen,
- Fig. 7:: einen Schnitt gemäss der Ebene VII-VII in Fig. 6 in einer gespannten Stellung des Austragkolbens, und
- Fig. 8:: einen Schnitt gemäss der Ebene VIII-VIII in Fig. 6 in einer entspannten Stellung des Austragkolbens.

Im folgenden werden bei den Ausführungsbeispielen Doppelspritzen dargestellt und beschrieben, doch kann es sich auch um Mehrfachspritzen oder Mehrfachkartuschen mit mehr als zwei Vorratsbehältern handeln, wobei dann der Austragkolben hierfür angepasst ist. Die Begriffe "vorne" und "hinten" beziehen sich auf den Spritzenauslass, der vorne ist.

Fig. 1 zeigt eine erste Ausführungsform einer erfindungsgemässen Austragvorrichtung 1 für eine Doppelspritze, mit einem Austragkolben 2, einem Führungsteil 3, einem Federelement 4 als Druckmedium, einem Handhabungsteil 5 und einer Arretierhülse 6.

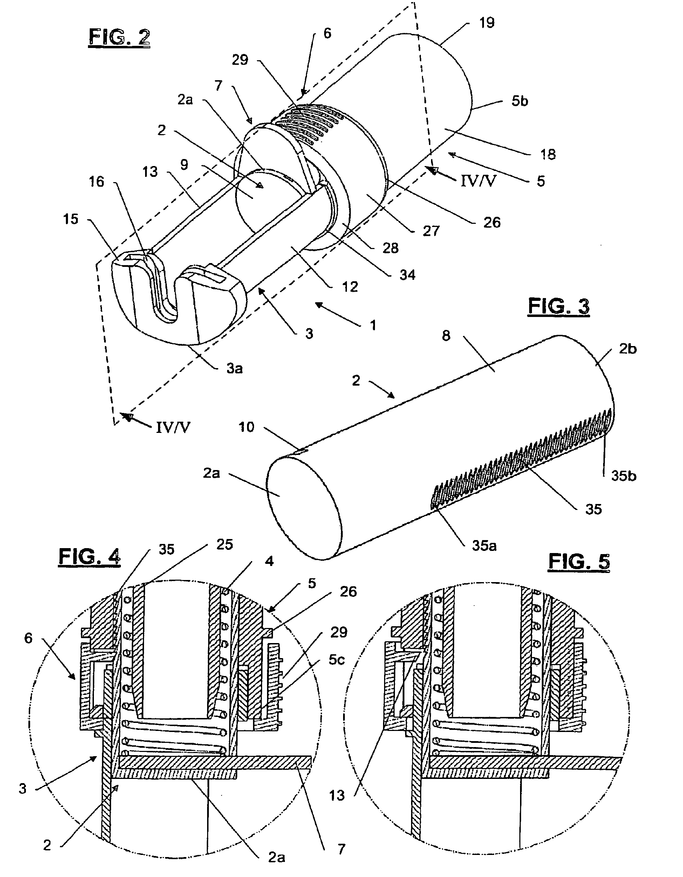

Der Austragkolben 2 gemäss den Figuren 1 und 3 weist die Form eines Hohlzylinders 8 auf, der an seinem vorderen Ende 2a durch eine Stösselauflage 9 abgeschlossen und an seinem hinteren Ende 2b offen ist. Beim vorderen Ende 2a der äusseren Mantelfläche des Austragkolbens 2 ist eine Fingerauflage 7 angeordnet, deren Unterseite entlang eines Teilumfangs des Hohlzylinders 8 verläuft und deren ebene Oberfläche sich im Wesentlichen senkrecht zur Mantelfläche des Hohlzylinders 8 erstreckt. Die Fingerauflage 7 ist in einen Aufnahmeschlitz 10 des Hohlzylinders 8 einsteckbar und darin fixierbar, wie in Fig. 1 näher ersichtlich und in der nachfolgenden Beschreibung genauer erläutert ist. Entlang der Unterseite des Austragkolbens 2 ist eine sägezahnähnliche Arretierverzahnung 35 vorgesehen. Die Arretierverzahnung 35 erstreckt sich über eine Distanz, welche dem Gesamthub des Austragkolbens entspricht. In der dargestellten Position des Austragkolbens 2 befindet sich die vorderste Arretierverzahnungslücke 35a im Axialbereich des Arretierzahns 30. Die Innenseite des Arretierzahns 30 weist eine zu den Verzahnungslücken äquivalente Form auf, so dass der Arretierzahn 30 formschlüssig in jede der Verzahnungslücken 35 einrasten kann.

Wie aus Fig. 1 hervorgeht, besteht das Führungsteil 3 im Wesentlichen aus einem Hohlzylinder 12 mit einem offenen hinteren Ende 3b, in welchem der Austragkolben 2 aufnehmbar ist. An seiner Manteloberseite weist der Hohlzylinder 12 eine Durchtrittsöffnung 13 auf, welche sich in Längsrichtung über einen Grossteil des Führungsteils 3 erstreckt und deren Breite im Wesentlichen dem Anschlussbereich der Fingerauflage 7 am Austragkolben 2 entspricht und im Weiteren der Aufnahme des Doppelstössels 55 dient. Dadurch ist eine Axialbewegung des Austragkolbens 2 innerhalb des Führungsteils 3 möglich, wobei die Fingerauflage 7 über die Durchtrittsöffnung 13 hinausragt. Durch die Breite des Querstegs 14 ist entlang eines Teils des Aussenumfangs des Führungsteils 3 eine vordere Anschlagschulter 34 für die Arretierhülse 6 definiert.

Die Kolbenbewegung innerhalb des Führungsteils 3 ist durch einen Aufnahmeflansch für Spritze 15 am vorderen Ende 3a des Führungsteils 3 begrenzt. Dieser Aufnahmeflansch für Spritze 15 ist als Querwand ausgebildet, die sich beidseitig über den Zylinderdurchmesser hinaus erstreckt und in ihrem Mittelbereich eine U-förmige Ausnahme aufweist, durch welche eine Doppelspritze 50 und deren Doppelstössel 55 führbar ist. Entlang der Oberkante des Aufnahmeflansches 15 ist eine Einstecköffnung 16 für den Spritzenflansch einer Doppelspritze vorhanden, wie aus Fig. 1 ersichtlich ist. Somit ist beim Beaufschlagen des Doppelstössels 55 die vordere Endposition des Austragkolbens 2 innerhalb des Führungsteils 3 am Anschlagpunkt des Doppelstössels innerhalb der Doppelspritze erreicht.

Das Federelement 4 ist durch eine zylindrisch gewickelte Druckfeder gebildet, deren vorderes Ende 4a derart im Innenraum des Hohlzylinders 8 angeordnet ist, dass ihr hinteres Ende 4b aus dem offenen hinteren Ende 2b des Austragkolbens 2 hinausragt.

In Fig. 1d ist eine zusammengebaute erfindungsgemässe Vorrichtung mit einer Doppelspritze 50 und einem Mischer 53 dargestellt. Die Doppelspritze wird mit einem Doppelstössel 55 beaufschlagt und weist ferner einen Rückhalteflansch 54 und eine Druckplatte 57 auf.

Gemäss Fig. 1a weist das Handhabungsteil 5 ebenfalls eine im Wesentlichen zylindrische Gestalt 18 auf, deren vorderes Ende 5a offen ist und deren hinteres Ende 5b durch eine Endwand 19 abgeschlossen ist. Der hohlförmige Innenraum des Handhabungsteils 5 erweitert sich im vorderen Bereich an einem umfänglich entlang der Innenwandung verlaufenden Anschlagbund 20..

Durch den stufenförmigen Anschlagbund 20 ist ein vorderer Aufnahmebereich 33 des Handhabungsteils 5 definiert, welcher zur Aufnahme des hinteren Endabschnitts 3c des Führungsteils 3 ausgebildet ist, so dass dessen hinteres Ende 3b an den stufenförmigen Anschlagbund 20 anschliesst. An zwei gegenüberliegenden Seiten der Innenwandung des Aufnahmebereichs 33 befindet sich jeweils ein Positioniernocken 21a, b, welche sich ausgehend von den stufenförmigen Anschlagbund 20 in vorderer Richtung erstrecken.

Demgemäss sind im hinteren Endabschnitt 3c des Führungsteils 3 zwei entsprechende Positionierschlitze 22a, b vorhanden, wodurch beim Einschieben des Führungsteils 3 in das Handhabungsteil 5 dessen korrekte Orientierung sichergestellt wird. An zwei gegenüberliegenden Seiten auf der Oberfläche des hinteren Endabschnitts 3c des Führungsteils 3 ist jeweils eine Schnappnase 23a, b angeordnet. Entsprechende Schnapprillen 24a, b sind entlang der Innenwandung des Aufnahmebereichs 33 vorhanden. Dadurch wird beim Zusammenfügen der Bauteile 3, 5 eine unlösbare Schnappverbindung zwischen den Schnappnasen 23a, b und den Schnapprillen 24a, b verwirklicht.

Eine zylindrische Federführung 25 ist im Zentralbereich des hohlförmigen Innenraums des Handhabungsteils 5 angeordnet und erstreckt sich in axialer Richtung im Wesentlichen über dessen Gesamtlänge, wobei sie innenseitig in die Endwand 19 mündet. Dabei entspricht der Aussendurchmesser der zylindrischen Federführung 25 im Wesentlichen dem Innendurchmesser des Federelements 4. Dadurch ist das hintere Ende 4b des Federelements 4 über die zylindrische Federführung 25 an die Innenseite der Endwand 19 führbar, wodurch beim Zusammenfügen der Bauteile 2, 3, 4 und 5 das Federelement 4 zusammengedrückt wird.

Eine hintere Anschlagschulter 26 ist entlang der zylindrischen Oberfläche 18 umfänglich auskragend hinter dem Aufnahmebereich 33 angeordnet. Dabei befindet sich die Anschlagschulter am vorderen Ende des Endabschnitts 5c des Handhabungsteils 5.

Gemäss den Figuren 1b und 1c umfasst die Arretierhülse 6 einen ringförmigen Hülsenmantel 27, der über den vorderen Endabschnitt 5c des Handhabungsteils 5 aufschiebbar ist. Dabei ist im Vergleich zum Aussendurchmesser des Endabschnitts 5c ein leicht ovalförmiger Innendurchmesser des Hülsenmantels 27 vorgesehen, dessen grösste Abmessung sich zwischen Arretierzahn und Fingerauflage befindet und eine entsprechende Verschiebung ermöglicht. Die vordere Stirnseite 28 der Arretierhülse 6 liegt nach deren Montage an der vorderen Anschlagschulter 34 des Führungsteils 3 an. Das hintere Ende 6b der Arretierhülse 6 grenzt dann an die hintere Anschlagschulter 26 des Handhabungsteils 5.

Entlang der Innenwandung des Hülsenmantels 27 ist ein Arretierzahn 30 angeordnet, der sich radial nach innen erstreckt. Einem dem Arretierzahn 30 entsprechende seitliche Durchgangsöffnung 32 ist im vorderen Endabschnitt 5c des Handhabungsteils 5 vorgesehen. Infolge der elliptischen Form der Innenabmessung der Arretierhülse und der zylindrischen Oberfläche 5c ist ein Heranführen des Arretierzahns 30 an die seitliche Durchgangsöffnung 32 beim Zusammenfügen der Bauteile 5, 6 und ein anschliessendes Einführen des Arretierzahns 30 in die seitliche Durchgangsöffnung 32 ermöglicht, so dass der Arretierzahn 30 in den hohlförmigen Innenraum des Handhabungsteils 5 hineinragt.

Gemäss Fig. 1c ist auf der dem Arretierzahn 30 gegenüberliegenden Seite der Innenwandung des Hülsenmantels 27 ein elastisches Druckelement 31 angeordnet. Das Druckelement 31 sorgt für eine Kraftbeaufschlagung der Arretierhülse 6 in Gegenrichtung bezüglich des Arretierzahns 30, um diesen mit der Arretierverzahnung in Eingriff zu bringen. Hülsenaussenseitig ist dem elastischen Druckelement 31 eine Fingerauflage 29 zugeordnet. Durch Druck auf diese Fingerauflage wird das Druckelement 31 und somit die Arretierhülse 6 mit dem Arretierzahn 30 innerhalb des Radialspiels aus der Arretierverzahnung 35 auf der Unterseite des Austragkolbens 2 gezogen und somit der Austragkolben freigegeben.

In Fig. 4 und 5 ist die Austragvorrichtung 1 in einer Schnittansicht gemäss den Ebenen IV-IV und V-V in Fig. 2 dargestellt. In der in Fig. 4 gezeigten Verschlussstellung der Arretierhülse 6 befindet sich die Fingerauflage 29, in der durch das Druckelement beaufschlagten und somit vom vorderen Endabschnitt 5c des Handhabungsteils 5 innerhalb des Radialspiels entfernten Position. Dabei ragt die Innenseite des Arretierzahns 30 durch die Durchgangsöffnung 32 in den Innenraum des Handhabungsteils 5. Dadurch rastet der Arretierzahn 30 in jeweils eine Verzahnungslücke der entlang der Innenwandung des Handhabungsteils 5 verlaufenden Arretierverzahnung 35 des Austragkolbens 2. In der hier dargestellten hinteren Endposition des Austragkolbens 2 rastet der Arretierzahn 30 in die vordere Verzahnungslücke 35a. Durch die so gebildeten Arretiermittel ist der Austragkolben 2 in seiner Axialposition bezüglich dem Handhabungsteil 5 und entgegen der Kraftbeaufschlagung durch das Federelement 4 festgehalten.

In der in Fig. 5 gezeigten Auslösestellung der Arretierhülse 6 befindet sich die Fingerauflage 29 in einer über eine manuelle Druckeinwirkung hervorgerufenen gegen den vorderen Endabschnitt 5c des Handhabungsteils 5 innerhalb des vorgegebenen Spiels verschobenen Position. Dadurch ist die sägezahnförmige Innenseite des Arretierzahns 30 aus dem Innenraum des Handhabungsteils 5 nach aussen geführt, wodurch ein Lösen der Arretiermittel erfolgt.

Beim Aufheben der manuellen Druckeinwirkung auf die Fingerauflage 29 wird die Arretierhülse 6 durch die Federwirkung des Druckelements 31 in die in Fig. 4 gezeigte Verschlussstellung zurückgeführt. Dadurch wird ein Einrasten in der nächstliegenden Verzahnungslücke bewirkt. Somit ist durch die Arretierverzahnung 35 ein Einrasten des Austragkolbens 2 am Handhabungsteil 5 nach Bedarf bei verschiedene Kolbenpositionen möglich.

Eine manuelle Rückführung des Austragkolbens 2 nach hinten ist aus jeder Kolbenposition möglich, indem Druck auf die Fingerauflagefläche 11 der Fingerauflage 7 ausgeübt wird, denn die keilförmige Ausbildung des Arretierzahns 30 bzw. der Arretierverzahnung 35 erlaubt ein Loslösen des Arretierzahns 30 nacht hinten aus einer Verzahnungslücke.

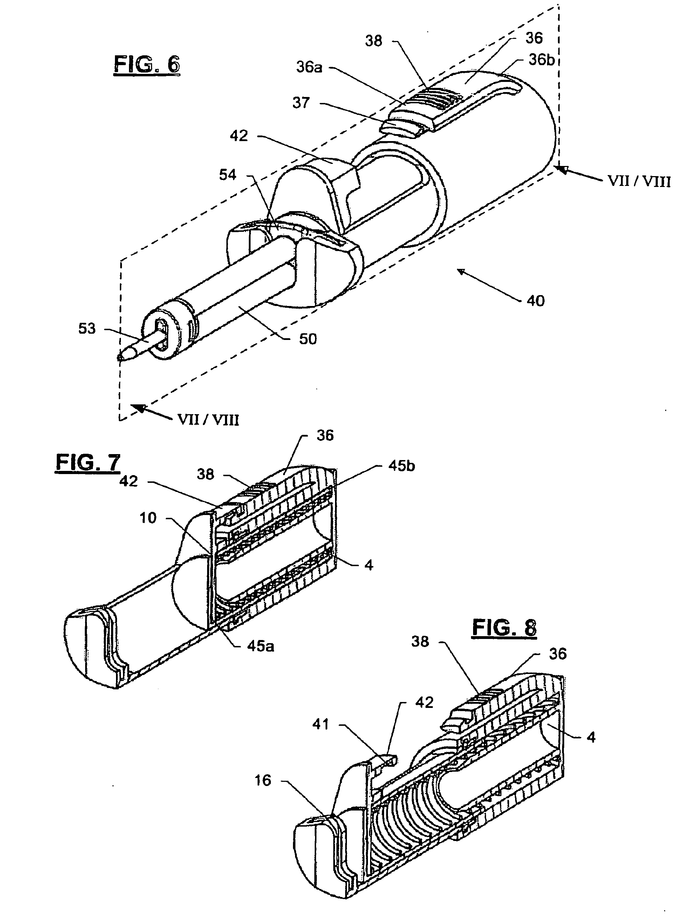

Fig. 6 zeigt ein zweites Ausführungsbeispiel einer erfindungsgemässen Austragvorrichtung 40 in einer perspektivischen Darstellung. Diejenigen Merkmale, welche dem ersten Ausführungsbeispiel entsprechen, sind dabei mit dem gleichen Bezugszeichen versehen. Weiterhin ist eine Doppelspritze 50 dargestellt, welche zur Verwendung mit der Austragvorrichtung 1, 40 geeignet ist.

Diese Variante entspricht im Wesentlichen dem ersten Ausführungsbeispiel, wobei der Hauptunterschied in einem vereinfachten Arretier- und Aulösemechanismus besteht und der Austragvorgang nicht unterbrochen werden kann.

Die Vorrichtung 40 umfasst eine federnde Fingerauflage 36, deren hinteres Ende 36b am Handhabungsteil 5 im Bereich des hinteren Endes 5b starr befestigt ist und sich nach vorne entlang der zylindrischen Oberfläche 18 bis hin zum vorderen Ende 5a des Handhabungsteils 5 fortsetzt. Am vorderen Ende 36a der Fingerauflage 36 ist ein Arretierzahn 37 angeordnet. Dabei übernimmt die Fingerauflage mit Arretierzahn im Wesentlichen die Aufgabe der Arretierhülse 6 in der ersten Ausführungsform 1 der Austragvorrichtung. In dieser Ausführung ist der Arretierzahn 37 im Gegensatz zum ersten Ausführungsbeispiel nach aussen gerichtet.

Dem Arretierzahn 37 ist eine entsprechende Arretiernut 41 am Austragkolben 2 vorgesehen. Der Austragkolben 2 weist am vorderen Ende einen Aufnahmeschlitz 10 auf, in welchem die Fingerauflage 7 steckbar ist. Die Fingerauflage weist einen Arretiersupport 42 auf, der schirmförmig über dem Hohlzylinder 8 des Austragkolbens 2 verläuft. Auf der Unterseite von Arretiersupport 42 ist die Arretiernut 41 ausgebildet, siehe Fig. 7.

Bei Druckeinwirkung auf die Druckfläche 38 der Fingerauflage 36 wird der Arretierzahn 37 hinuntergedrückt und rastet aus der Arretiernut 41 aus. Durch die als Federarm ausgebildeten Fingerauflage 36 wird der Arretierzahn 37 nach Aufheben der manuellen Druckeinwirkung in seine Ursprungsposition zurückgeführt, wobei durch die Keilform des Arretierzahns 37 bei einer Wiederverwendung des Gerätes zu einem späteren Zeitpunkt ein erneutes Einrasten in der hinteren Position gewährleistet ist.

Die Figuren 7 und 8 zeigen eine perspektivische Schnittansicht des Führungsteils 2 gemäss den Schnittebenen VII-VII und VIII-VIII in Fig. 6 in der gespannten und ausgelösten Stellung.

Aus der Beschreibung der Ausführungsbeispiele sind dem Fachmann zahlreiche Abwandlungen zugänglich, ohne den Schutzbereich der Erfindung zu verlassen, der durch die Ansprüche definiert ist. So ist auch bei externen Arretiermitteln gemäss dem zweiten Ausführungsbeispiel eine Arretierverzahnung denkbar. Dadurch ist durch einfaches Aufheben der manuellen Druckeinwirkung eine Fixierung des Austragkolbens in einem Arretiermittel zu jedem Zeitpunkt des Austragens möglich. Weiterhin sind auch Arretiermittel denkbar, die einen Arretierzahn am Austragkolben 2 und eine Arretierverzahnung am Handhabungsteil 5 umfassen. Ferner ist denkbar, ein externes Arretiermittel durch einen Arretierzahn an einer Arretierhülse oder ein internes Arretiermittel über einen Arretierbügel am Handhabungsteil vorzusehen.

Ausserdem kann als Druckmedium anstatt einer Druckfeder eine Druckluftpatrone oder dergleichen Druckmedium verwendet werden, um das Selbst-Austragen zu erleichtern.

## Patentansprüche

1. Vorrichtung zur Aufnahme und zum Austragen einer Spritze (50), mit einem mit einem Druckmedium (4) beaufschlagten Austragkolben (2), einem Handhabungsteil (5) das zur Aufnahme des Austragkolbens ausgebildet ist, sowie über eine manuelle Druckeinwirkung auf den Handhabungsteil lösbare Arretiermittel (30, 37), die am Handhabungsteil angeordnet sind und in Wirkverbindung mit Arretiermitteln (35, 41) am Austragkolben stehen, **dadurch gekennzeichnet, dass** am Handhabungsteil (5) ein Führungsteil (3), angeschlassen ist, innerhalb welchem der Austragkolben (2) verschiebbar gelagert ist und das auslassseitig einen Aufnahmeflansch (15) zur Befestigung der Spritze (50) an ihrem Rückhalteflansch (54) aufweist, wobei die Spritze eine Doppelspritze oder eine Mehrfachspritze oder Mehrfachkartusche mit mehr als zwei Vorratsbehältern ist und der Austragkolben (2) ausgebildet ist, auf den Mehrfachstössel (55) der Spritze (50) zu wirken.

2. Vorrichtung nach Anspruch 1, **dadurch gekennzeichnet, dass** das Druckmedium ein Federelement (4) ist, das im Innern des Austragkolbens (2) angeordnet ist und diesen überragt.

3. Vorrichtung nach Anspruch 1 oder 2, **dadurch gekennzeichnet, dass** die Arretiermittel (6) einen Arretierzahn (30, 37) am auslassseitigen Ende des Handhabungsteils (5) und mindestens eine mit dem Arretierzahn kooperierende Arretierverzahnung (35, 41) am Austragkolben (2) enthalten.

4. Vorrichtung nach Anspruch 3, **dadurch gekennzeichnet, dass** der Austragkolben (2) eine Mehrzahl von Arretierverzahnungen (35, 41) aufweist.

5. Vorrichtung nach einem der Ansprüche 1 bis 4, **dadurch gekennzeichnet, dass** die Arretiermittel eine Arretierhülse (6) enthalten, die am auslassseitigen Ende des Handhabungsteils (5) angeordnet ist und im Innern einen mit der Arretierverzahnung (35) kooperierenden Arretierzahn (30) aufweist.

6. Vorrichtung nach Anspruch 5, **dadurch gekennzeichnet, dass** die Arretierhülse (6) an einem dem Arretierzahn (30) gegenüberliegenden Innenbereich ein elastisches Druckelement (31) aufweist, um den Arretierzahn(30) in eine Lücke der Arretierverzahnung zu drücken derart, dass die Arretiermittel durch manuelle Druckeinwirkung auf der Aussenseite dieses dem Arretierzahns gegenüberliegenden Bereichs der Arretierhülse lösbar sind.

7. Vorrichtung nach einem der Ansprüche 1 bis 4, **dadurch gekennzeichnet, dass** die mindestens eine Arretierung (41) am Ende eines federnden Arretiersupports (36, 42) angeordnet ist, der sich längs des Austragkolbens (2) erstreckt.

8. Vorrichtung nach einem der Ansprüche 1 bis 7, **dadurch gekennzeichnet, dass** der Austragkolben (2) an seinem auslassseitigen Ende eine Fingerauflage (7) zur manuellen Überführung des Austragkolbens (2) von einer jeweils vorderen in eine hintere Stellung aufweist.

9. Vorrichtung nach einem der Ansprüche 1 bis 8, **dadurch gekennzeichnet, dass** das Handhabungsteil (5) in seinem Innenraum eine im Wesentlichen zylindrisch ausgebildete und axial verlaufende Federführung (25) aufweist.

10. Vorrichtung nach einem der Ansprüche 1 bis 9, **gekennzeichnet durch** mindestens zwei gegenüberliegende Positionierschlitze (22a, b) am hinteren Endabschnitt (3c) des Führungsteils (3) und entsprechende Positioniernocken (21a, b) an der Innenwandung des Handhabungsteils (5).

11. Vorrichtung nach einem der Ansprüche 1 bis 10, **dadurch gekennzeichnet, dass** der am auslassseitigen Ende (3a) des Führungsteils (3) vorhandene Aufnahmeflansch (15) für eine Mehrfachspritze (50) oder -kartusche kodiert ist, in dem die Mehrfachspritze (50) oder -kartusche gerichtet fixierbar ist.

12. Anordnung zur Applikation medizinischer Präparate, **gekennzeichnet durch** eine Vorrichtung (1, 40) nach einem der Ansprüche 1 bis 11 und **durch** eine Mehrfachspritze (50), die in die Vorrichtung (1, 40) einsteckbar ist.

## Claims

1. Device for receiving and dispensing a syringe (50), comprising a dispensing piston (2) that is actuated by a pressure medium (4), a handling portion (5) that is configured to receive the dispensing piston, as well as locking means (30, 37) that are releasable by applying manual pressure to the handling portion and are arranged on the handling portion and operatively connected to locking means (35, 41) provided on the dispensing piston, **characterised in that** the handling portion (5) comprises a guide portion (3) within which the dispensing piston (2) is displaceably supported and whose outlet side has a receiving flange (15) for fastening the syringe (50) by its retaining flange (54), the syringe being a double syringe or a multiple syringe or multiple cartridge having more than two storage containers and the dispensing piston (2) being configured so as to act upon the multiple plunger (55) of the syringe (50).

2. Device according to claim 1, **characterised in that** the pressure medium is a spring element (4) that is arranged in the interior of the dispensing piston (2) and projects therefrom.

3. Device according to claim 1 or 2, **characterised in that** the locking means (6) comprise a locking catch (30, 37) at the outlet side end of the handling portion (5) and at least one locking toothing (35, 41) on the dispensing piston (2) that cooperates with the locking catch.

4. Device according to claim 3, **characterised in that** the dispensing piston (2) has a plurality of locking toothings (35, 41).

5. Device according to any one of claims 1 to 4, **characterised in that** the locking means comprise a locking sleeve (6) that is arranged at the outlet side end of the handling portion (5) and provided in its interior with a locking catch (30) which cooperates with the locking toothing (35).

6. Device according to claim 5, **characterised in that** an inner area of the locking sleeve (6) opposite the locking catch (30) is provided with an elastic pressure element (31) for pushing the locking catch (30) into a tooth space of the locking toothing such that the locking means are releasable by applying manual pressure to the outside of this area of the locking sleeve opposite the locking catch.

7. Device according to any one of claims 1 to 4, **characterised in that** the at least one locking means (41) is arranged at the end of an elastic locking support (36, 42) extending in the longitudinal direction of the dispensing piston (2).

8. Device according to any one of claims 1 to 7, **characterised in that** the dispensing piston (2) is provided at its outlet side end with a finger rest (7) for manually transferring the dispensing piston (2) from a respective forward to a rearward position.

9. Device according to any one of claims 1 to 8, **characterised in that** the handling portion (5) comprises in its interior an essentially cylindrically shaped and axially extending spring guide (25).

10. Device according to any one of claims 1 to 9, **characterised by** at least two opposed positioning slots (22a, b) at the rear end section (3c) of the guide portion (3) and corresponding positioning cams (21a, b) on the inner wall of the handling portion (5).

11. Device according to any one of claims 1 to 10, **characterised in that** the receiving flange (15) provided at the outlet side end (3a) of the guide portion (3) is coded for a multiple syringe (50) or cartridge for affixing the multiple syringe (50) or cartridge in a directional manner.

12. Arrangement for the application of medical preparations, **characterised by** a device (1, 40) according to any one of claims 1 to 11 and by a multiple syringe (50) that is insertable into the device (1, 40).

## Revendications

1. Dispositif destiné à recevoir une seringue (50) et distribuer son contenu, comprenant un piston de distribution (2) actionné par un moyen de pression (4), une partie de maniement (5) adaptée à recevoir le piston de distribution ainsi que des moyens d'arrêt (30, 37) libérables par l'application d'une pression manuelle sur la partie de maniement et fonctionnellement reliés à des moyens d'arrêt (35, 41) sur le piston de distribution, **caractérisé en ce que** la partie de maniement (5) comporte un élément de guidage (3) dans lequel est logé le piston de distribution (2) de manière déplaçable et qui présente du côté de sortie un flasque de réception (15) pour la fixation de la seringue (50) par son flasque de retenue (54), la seringue étant une seringue double ou une seringue multiple ou cartouche multiple ayant plus que deux réservoirs et le piston de distribution (2) étant adapté à agir sur le poussoir multiple (55) de la seringue (50).

2. Dispositif selon la revendication 1, **caractérisé en ce que** le moyen de pression est un élément à ressort (4) qui est agencé à l'intérieur du piston de distribution (2) et dépasse de celui-ci.

3. Dispositif selon la revendication 1 ou 2, **caractérisé en ce que** les moyens d'arrêt (6) comprennent une dent d'arrêt (30, 37) du côté de sortie de la partie de maniement (5) et au moins une denture d'arrêt (35, 41) sur le piston de distribution (2) qui coopère avec ladite dent d'arrêt.

4. Dispositif selon la revendication 3, **caractérisé en ce que** le piston de distribution (2) comporte une pluralité de dentures d'arrêt (35, 41).

5. Dispositif selon l'une quelconque des revendications 1 à 4, **caractérisé en ce que** les moyens d'arrêt comprennent une douille d'arrêt (6) agencée à l'extrémité du côté de sortie de la partie de maniement (5) et comportant à l'intérieur une dent d'arrêt (30) qui coopère avec ladite denture d'arrêt (35).

6. Dispositif selon la revendication 5, **caractérisé en ce que** la douille d'arrêt (6) comporte dans une zone intérieure opposée à la dent d'arrêt (30) un élément de pression (31) élastique afin d'enfoncer la dent d'arrêt (30) dans un espace dans la denture d'arrêt de telle manière que les moyens d'arrêt sont libérables par l'application d'une pression manuelle sur le côté extérieur de cette zone de la douille d'arrêt opposée à la dent d'arrêt.

7. Dispositif selon l'une quelconque des revendications 1 à 4, **caractérisé en ce que** ledit au moins un moyen d'arrêt (41) est agencé à l'extrémité d'un support d'arrêt (36, 42) élastique qui s'étend le long du piston de distribution (2).

8. Dispositif selon l'une quelconque des revendications 1 à 7, **caractérisé en ce que** le piston de distribution (2) comporte à son extrémité du côté de sortie un appuie-doigt (7) pour amener le piston de distribution (2) d'une position respective à l'avant vers une position arrière.

9. Dispositif selon l'une quelconque des revendications 1 à 8, **caractérisé en ce que** la partie de maniement (5) présente dans son intérieur un élément de guidage (25) du ressort de forme essentiellement cylindrique s'étendant en direction axiale.

10. Dispositif selon l'une quelconque des revendications 1 à 9, **caractérisé par** au moins deux fentes de positionnement (22a, b) opposées dans la partie d'extrémité (3c) arrière de l'élément de guidage (3) et des cames de positionnement (21a, b) correspondantes sur la paroi intérieure de la partie de maniement (5).

11. Dispositif selon l'une quelconque des revendications 1 à 10, **caractérisé en ce que** le flasque de réception (15) agencé à l'extrémité (3a) du côté de sortie de l'élément de guidage (3) est codé pour une seringue (50) ou cartouche multiple et permet la fixation directionnelle de la seringue (50) ou cartouche multiple.

12. Agencement pour l'application de préparations médicales, **caractérisé par** un dispositif (1, 40) selon l'une quelconque des revendications 1 à 11 et par une seringue multiple (50) insérable dans ledit dispositif (1, 40).
